(19) Europäisches Patentamt
European Patent Office
Office européen des brevets

(11) Publication number: 0 135 125
A2

# (12) EUROPEAN PATENT APPLICATION

(21) Application number: 84109541.7

(22) Date of filing: 10.08.84

(51) Int. Cl.[4]: G 01 N 24/02
G 01 N 24/08

(30) Priority: 12.08.83 US 522526

(43) Date of publication of application:
27.03.85 Bulletin 85/13

(84) Designated Contracting States:
BE DE FR GB IT LU NL

(71) Applicant: E.I. DU PONT DE NEMOURS AND COMPANY
1007 Market Street
Wilmington Delaware 19898(US)

(72) Inventor: Fossel, Eric Thor
66 Priscella Road
Chestnut Hill Massachusetts 02167(US)

(74) Representative: von Kreisler, Alek, Dipl.-Chem. et al,
Deichmannhaus am Hauptbahnhof
D-5000 Köln 1(DE)

## (54) NMR imaging utilizing chemical shift reagents.

(57) The subject invention is a process for NMR diagnostic imaging comprising introducing into a mammalian body a chemical shift reagent in an amount capable of altering the resonance frequency of atomic nuclei of diagnostic interest, and thereafter depicting the resulting shift in resonance frequency on a composite NMR signal spatial representation.

EP 0 135 125 A2

# TITLE

# NMR IMAGING UTILIZING CHEMICAL SHIFT REAGENTS

## BACKGROUND

The subject invention relates to the field of diagnostic imaging, more particularly to nuclear magnetic resonance (NMR) imaging.

For years, the medical community has relied upon the principles of differential x-ray absorption to construct diagnostic images of the mammalian body. Recently, a technique known as computerized axial tomography has been developed whereby absorption data of x-rays projected from different directions is accumulated and reconstructed to result in cross-sectional views of the body organ being imaged. This technique however, although providing useful anatomical data, does not provide information pertaining to the physiological state of organs of interest. X-ray imaging, of course, also has the disadvantage of exposing the patient to potentially harmful ionizing radiation.

NMR spectroscopy has been used for decades as an analytical tool to determine the distribution of NMR sensitive atomic nuclei in samples of various materials. Certain atomic nuclei exhibit an inherent net nuclear spin. These nuclei, in an unmolested state, point in random directions. When placed in an external magnetic field, however, the nuclei align either parallel or anti-parallel to the external magnetic field and precess at a frequency F (Larmour frequency) which is dependent upon the strength of the magnetic field and the gyromagnetic ratio of the nucleus. When these nuclei are subjected to an applied electromagnetic pulse, a resonant effect is created which results in the nuclei changing their alignment relative to the external magnetic field. After the application of the pulse, the nuclei return to their original alignment. The time required for the spin system to return to thermal equilibrium with its surroundings is known as the spin-lattice relaxation time ($T_1$), and the time required for the free induction signal to decay is known as the spin-spin relaxation time ($T_2$). Each family of like atomic nuclei with an

0435125

inherent net spin possesses characteristic F, $T_1$ and $T_2$ values resulting in a particular NMR signal for the nuclei. Characterization of NMR signals, or "peaks" produced by differing resonance frequencies for various atomic nuclei has allowed NMR spectrophotometers to differentiate among such nuclei.

It has been discovered that the same principles which make NMR spectroscopy useful as an analytical tool, when used in conjunction with heretofore known chemical shift imaging methods, also makes NMR useful as a diagnostic imaging modality. The application of NMR concepts to diagnostic imaging, however, requires that NMR imaging apparatus be able to differentiate resonance frequencies of similar atomic nuclei that are, for example, located both in healthy and unhealthy bodily tissue. In certain instances, the interaction of these nuclei with unhealthy tissue differs sufficiently from their interactions with healthy tissue that a diagnostically useful image can be obtained without the use of pharmacologic enhancement. More often, however, it is necessary to introduce into either healthy or unhealthy tissue a paramagnetic agent that reduces the $T_1$ and $T_2$ values of the nuclei in that tissue. The NMR signal for the tissue hosting the paramagnetic agent, based on altered $T_1$ and $T_2$ values, will then be distinguishable from the NMR signal based on unaltered $T_1$ and $T_2$ values for the other tissue. For a more comprehensive discussion of NMR imaging in general see "NMR Imaging in Medicine", Sci. Amer., 246, pp 78-88 (1982). Often times, however, altering the relaxation times does not result in a sufficient increase in the signal to background ratio to be of unambiguous diagnostic value.

Another approach to differentiate resonance frequencies of similar atomic nuclei is to utilize one of the well known chemical shift imaging methods, for example, the Fourier 3-D imaging method disclosed by T.R. Brown, B.M. Kincaid and K. Ugurbil in "NMR Chemical Shift Imaging", Proc. Natl. Acad. Sci USA, 79, pp 3523-3526 (1980), hereby incorporated by reference, and the chemical shift imaging method known in the

art as back-projection reconstruction. These methods rely on chemical shifts inherent in the resonance frequencies imaged.

## SUMMARY OF THE INVENTION

The subject invention is an NMR diagnostic imaging process which utilizes conventional chemical shift imaging methods in conjunction with paramagnetic ions capable of inducing resonance frequency shifts.

More specifically, the subject invention is a process for NMR diagnostic imaging comprising introducing into a mammalian body a chemical shift reagent in an amount capable of altering the resonance frequency of atomic nuclei of diagnostic interest, and thereafter depicting the resulting shift in resonance frequency on a composite NMR signal spatial representation.

## DETAILED DESCRIPTION

NMR chemical shift reagents, when introduced to a tissue site of interest, alter the resonance frequencies which make up the magnetic resonance spectrum of certain nuclei in that particular tissue. When used in conjunction with conventional NMR shift imaging methods detailed above, the subject invention is utilized by localizing a chemical shift reagent in tissue of interest to shift the resonance frequency of the associated group of atomic nuclei of interest (e.g., aliphatic protons from the side chains of amino acids and protons of fatty acids of phospholipids in malignant tissue) to an area of the NMR spectrum where no NMR signal is generated from either normal or abnormal tissue. In other words, a new resonance intensity is created in tissue which is abnormal and a chemical shift image is created which images only this new intensity, thereby increasing the target to background NMR signal ratio to nearly infinity. Alternatively, if the agent which induces the shift is introduced into healthy tissue, the unhealthy tissue may be imaged at the unshifted resonance frequency. The intensity of the resonance of interest for each pixel in the imaging field is measured by NMR imaging apparatus and a composite NMR signal spatial representation is processed therefrom.

Chemical Shift Reagents: Reagents which alter resonance frequency include the metal ions Pr, Nd, Pm, Sm, Eu, Gd, Tb, Dy, Ho, Er, Tm, Yb and Lu. These reagents act by altering (either increasing or decreasing) the net magnetic field experienced by a given nuclei of interest, using its electron cloud to shield or deshield the nuclei. The net effect is to alter the resonance frequency of the affected nuclei as the resonance frequency is directly related to the field experienced by the nuclei. As described below, these shift reagents can be introduced into a patient as an uncomplexed inorganic salt, as a chelate complex, and/or bound to a target seeking biologically active molecule. Preferred shift reagents are Pr, Nd, Pm, Sm, Tb, Dy, Ho, Er, Tm, Yb and Lu, and most preferred are Tb, Tm, Pr, Yb, and Lu due to favorable electron cloud interaction between these particular reagents in their chelated form, and the nuclei of interest.

Chelators: It may in certain circumstances be desirable to complex the chemical shift reagents described above with chelating agents for introduction into a patient. For example, some chemical shift reagents may exhibit toxicity that can be reduced or eliminated through complexation of the shift reagent by a chelator.

Also, chelates may be more effective in shifting the resonance frequency of atomic nuclei due to configurational considerations. That is, the lifetime of the interaction between a chelated chemical shift reagent and the nuclei which are being affected is often times longer than if the chemical shift reagent is uncomplexed.

Chelators useful in the practice of the subject invention include those compounds that bind one or more metal ions, such chelators generally well known in the art. Examples of such chelators are EDTA and more complex analogs of EDTA which have more carboxyl groups per molecule, phosphonates, polyphosphates, polyphenols, cryptates and crown ethers.

More specifically, as disclosed in Australian Patent Application 86 330/82 published January 27, 1983 subsequent to

the subject invention, paramagnetic ion chelators include those aminopolycarboxylic acids of the formulas:

(i) Nitrilo-triacetic acid (NTA)
(ii) N,N,N',N'-ethylenediamine-tetra-acetic acid (EDTA)
(iii) N-hydroxyethyl-N,N',N'-ethylenediamine-triacetic acid (HETDA)
(iv) N,N,N',N",N"-diethylenetriamine-penta-acetic acid (DTPA), and
(v) N-hydroxyethylimino-diacetic acid.

Also useful as metal chelators are the aminopolycarboxylic acids of the general formula

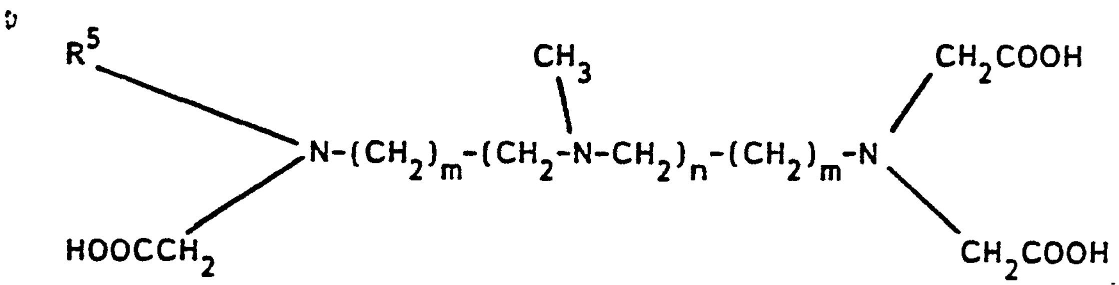

wherein m represents the integers 1 to 4,
n represents the integers 0 to 2, and
$R^5$ represents a saturated or unsaturated hydrocarbon radical having from 4 to 12 carbon atoms or the group $-CH_2-COOH$.

Also useful as chelators are amines corresponding to the general formula

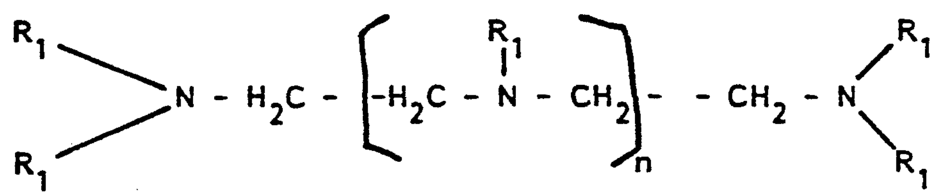

in which

$R_1$ and $R_1'$ are the same or different and represent hydrogen or an alkyl radical having 1 to 4 carbon atoms, and n represents the integers 0 to 4. Such amines are, for example, ethylendiamine, diethylenetriamine, triethylenetetramine, tetraethylene-pentamine and petaethylenehexamine.

Also useful as metal chelators are the macrocyclic compounds of the general formula

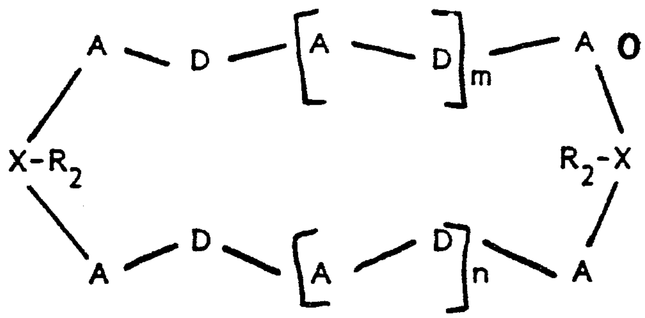

wherein

$R_2$ in each case represents a hydrogen atom, a hydrocarbon radical or an alkoxycarbonyl radical, or both radicals $R_2$ together form a group of the general formula

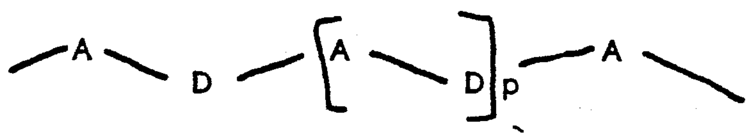

in which

A in each case represents a hydrocarbon radical,

X in each case represents a nitrogen or phosphorous atom, and

D in each case represents an oxygen or sulphur atom, a group of the formula $>$N-R (in which R represents a hydrogen atom or a hydrocarbon radical) or a hydrocarbon radical, with the proviso that at least two of the groups or atoms which D represents are oxygen or sulphur atoms or a group of the formula $>$N-R, and that when each $R_2$ is a hydrogen atom, a hydrocarbon radical or an alkoxycarbonyl radical and X is a nitrogen atom, one of the two radicals or atoms which D represents is an oxygen or sulphur atom and the other radical is an oxygen atom or a group of the formula $>$N-R, and m, n and p are integers from 0 to 5.

When A and D in this formula represent hydrocarbon radicals, these are preferably straight-chain or branched-chain

alkylene or alkenylene radicals having from 2 to 8 carbon atoms, for example ethylene, propylene, butylene and hexylene and their unsaturated analogues, also cycloalkylene and cycloalkenylene radicals, for example cyclopropylene cyclobutylene, cyclohexylene and cycloheptylene and their unsaturated analogues, as well as aromatic radicals, for example phenylene. The hydrocarbon radicals designated by $R_2$ are preferably straight-chain or branched-chain alkyl or alkenyl radicals having from 1 to 8 carbon atoms, also cycloalkyl, aralkyl and aryl radicals having from 3 to 12 carbon atoms. Of the alkoxycarbonyl radicals designated by $R_2$ those having up to 10 carbon atoms are preferred. Especially preferred are macrocyclic complex components which contain nitrogen and oxygen, for example 5,6-benzo-4,7,13,16,-21,24-hexaoxa-1,10-diazabiocyclo[8.8.8]hexacosane, 1,7,10,16-tetraoxa-4,13-diazacyclo-octadecane and 4,7,13,16,21,-24-hexaoxa-1,10-diazabiocyclo[8.8.8]-hexacosane.

Also suitable as chelators are diphosphonates of the general formula

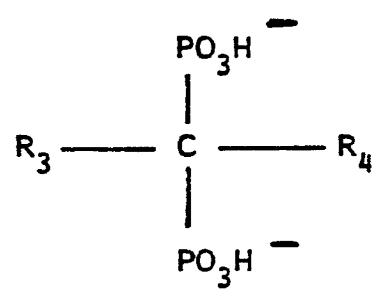

wherein

$R_3$ and $R_4$ are the same or different and represent a hydrogen atom, an alkyl radical having from 1 to 4 carbon atoms, a halogen atom, or an hydroxy, amino or $-CH_2-COOH-$ group.

<u>Biologically Active Molecules</u>: It may be highly desirable to conjugate the chemical shift reagents useful in the subject invention with a target seeking biologically active molecule (BAM) in order that the shift reagent localize in the tissue of interest. The chemical shift reagent may or may not be complexed by one of the chelators described above.

Examples of such BAM's include antibodies (especially monoclonal antibodies), Fab, Fab' and $F(ab')_2$ fragments of antibodies, hormones such as luteinizing hormones which bind to receptors in ovaries, quinuclidinyl benzylate which binds to muscarinic cholinergic receptors of the heart, estrogens, and neuropeptides.

Certain of these BAM's can be conjugated to shift reagents directly without imparing targeting properties using techniques well known in the art, or they can be conjugated through the chelators detailed above. If the BAM is an antibody or other protein/glycoprotein, it will contain functional groups such as -SH, $-NH_2$, -OH, -COOH, and/or -NHC(=NH) $NH_2$ for reaction with complimentary groups on a chelate molecule. If the BAM is a smaller drug or hormone that does not have available for conjugation such functional groups, these groups may be synthetically incorporated into the BAM for reaction with the chelate molecule. For a general discussion of such synthetic modification see Means and Feeney "Chemical Modification of Proteins", Holden-Day, Inc. (1971) hereby incorporated by reference.

Conditions for Shift Imaging: The concentration of shift reagent required in order to be effective in shifting resonance frequencies is preferably no more than .001 to .02 mM (millimoles per liter) although concentrations of 0.1 to 2.0 mM are readily attainable when utilizing a target seeking biologically active molecule as a carrier agent for the shift reagent. Concentrations in the low end of this range, ie., about .001mM are useful when utilizing well engineered chelators which may increase lifetime of contact or decrease the mean distance between the shift reagent and the nuclei whose resonance frequency is to be shifted. In addition the geometric relationship between the free electron orbital and the binding site for the nuclei to be shifted is important and greater effects can be achieved by optimization of this geometry.

Specifically, it has been found that as little as 1

millimolar shift reagent can shift proton resonances as much as 10 ppm (1ppm = $1 \times 10^{-4}$% shift) depending on the reagent; that 2 millimolar shift reagent can shift sodium resonances 1 to 20 ppm depending on the reagent; and that in extraordinary cases shifts of 50 ppm or more have been observed with millimolar shift reagent. A useful amount of shift is that which will bring the resonance shifted to a unique position in the NMR spectrum, or leave an unshifted peak of interest in a position in the spectrum, thus producing a target to background ratio approaching infinity. Often this is as little as 1 ppm but is probably not more than 3-4 ppm.

Shifts as small as one-half the line width of the resonance shifted are generally adequate for purposes of the subject invention. Line widths are usually not more than 1 ppm. However, larger shifts of tens or hundreds of ppm are acceptable though not necessarily better. Two cautions need be addressed. First, it is important to shift the resonance to a frequency where no resonance otherwise exists. Second, it is important to consider the fact that the amount of the shift is directly proportional to the concentration of shift reagent. Thus if the shift reagent concentration changes during the imaging time, positional uncertainty (blurring) will occur. Care must therefore be exercised in reaching a steady state in local concentration of contrast agent or in achieving short imaging times.

It will be apparent to those skilled in the art that the solutions of chemical shift agents, to be introduced into human patients, should be prepared in aseptic and steril, non-pyrogenic conditions. For intravenous introduction, such solutions should be parenterally compatable e.g., in physiological saline at physiological tolerable pH.

Those skilled in the art will recognize that homogeneity over the area imaged must be such that the resonance frequency upon which imaging is based, i.e. either a shifted or an unshifted resonance frequency, is distinguishable from background.

The magnetic field strength of the NMR imaging process employed also has an effect on the shifting of resonance

frequency. The minimum practical field strength required to utilize the concepts of the subject invention is about .01 Tesla (T), preferably greater than about 0.1T. Increasing magnetic field strength increases dispersion of chemical shift and increases NMR signal strength.

Increased chemical shift dispersion makes increasingly more resolvable resonances from tissue nuclear resonance spectra and makes the effect of chemical shift contrast reagents more dramatic. In addition, the increase in signal strength means either that the same image quality can be obtained in much shorter periods of time or that an image with much better resolution can be obtained in the same amount of time. Generally speaking, the maximum exposure time required to produce a useful diagnostic image is inversely proportional to the signal strength employed. Background noise does not change with an increase in field strength, therefore, the signal to noise ratio improves markedly with an increase in field strength.

NMR imaging instruments require site considerations as the environment must be kept free of fringing magnetic fields and the NMR imaging environment must be free from stray radio frequency, stray magnetic fields and from large nearby moving metal objects. This problem is important with all systems, but becomes more serious with magnets of high field strength (such as 1.5 T) and larger bore diameters (in the range of about 1 meter). Examples of conventional NMR imaging instrumentation include Foner Corporation's 3000 QED (0.3T) and Technicare Corporation's "Teslacon" NMR imagers. This instrumentation can be readily equipped to utilize one of the various chemical shift imaging methods known in the art such as Fourier 3-D imaging or back-projection reconstruction.

Instrumentation utilizing relatively low field strengths of about 0.12 to 0.6 T is capable of imaging proton, fluorine and sodium resonance frequencies. Higher field strength instruments have two advantages. First they can obtain proton images of far better resolution, or make images of the same resolution as lower field instruments much faster. For example, going from 0.3 to

1.5 T the same image can be obtained 25 times faster, or an image can be obtained with nearly 25 times the signal to noise in the same amount of time. Second, useful images of other nuclei such as Na-23, K-39 and Mg-25 can be obtained when chemical shift reagents are utilized in conjunction with these higher field strengths.

For example, Na-23 (100% naturally abundant) is relatively sensitive and easily observed at 1.5 T, and can be observed and imaged at fields as low as 0.35 T. Its main value is in tumor detection and detection of tissue ischemia. Malignant tumors have been known to concentrate sodium to levels as high as four times that in normal tissue and benign tumors. In the case of ischemia the example of myocardial ischemia is important. Following the onset of even mild (20-40% flow reduction) ischemia intracellular sodium concentrations increase rapidly and dramatically reaching 40-60 mM (an 8-12 fold increase) within minutes. This is much faster than redistribution of metabolites and dramatically faster than the release of creatine kinase. However the observation of intracellular sodium requires that a chemical shift reagent be utilized to induce a shift in the resonance frequency of extracellular sodium away from the resonance frequency of intracellular sodium. The unshifted intracellular peak can then be imaged.

Specific Indications:

Pulmonary and deep venous thrombosis: The use of shift reagents with NMR imaging can produce positive images of blood clots. A shift reagent such as Dy is linked to a protein having a high affinity for fibrin such as tissue plasminogen activator, and a resulting NMR image shows that the resonance frequency associated with the nuclei in the thrombus has been shifted to a position in the NMR spectrum where no resonance frequency previously existed.

Brain stroke: Shift reagents which are not conjugated to target specific molecules are distributed according to blood flow, such reagents are termed perfusion reagents, one example being a Dy/EDTA complex. Stroke can be detected very early

by monitoring such perfusion reagents in the brain. Those areas of the brain where resonance frequency shifts are not detected would indicate that those associated brain cells are being poorly perfused. The condition can then be corrected before cell damage becomes severe.

Myocardial Ischemia: At the onset of ischemia the normal trans-membrane distribution of sodium and potassium become markedly altered within the first thirty seconds. Increases in intracellular sodium of 5 fold or greater can be an extremely sensitive indicator of myocardial ischemia. In normal tissue myocardial sodium concentrations are about 5-8 mM. In totally dead tissue this rises to 145 mM. However, at the onset of ischemia this rises rapidly to 25-35 mM within very short time period. This change is rapidly reversed with reflow. In order to produce useful Na-23 images of the heart, two things are required. First, a perfusion shift reagent which alters the resonance frequency of only extracellular sodium (i.e., does not enter the cell) and second, NMR resolution of the heart utilizing gated images. Copending U.S.S.N. 279,721 (hereby incorporated by reference) discusses gating NMR imaging to bodily movements such as heart beats and in particular gating Na-23 images. Copending U.S.S.N. 264,100 (also incorporated herein by reference) discusses Na-23 imaging in general.

I CLAIM:

1. A process for NMR diagnostic imaging comprising introducing into a mammalian body a chemical shift reagent in an amount capable of altering the resonance frequency of atomic nuclei of diagnostic interest, and thereafter depicting the resulting shift in resonance frequency on a composite NMR signal spatial representation.

2. The process of claim 1 wherein the chemical shift reagent is selected from the group consisting of Pr, Nd, Pm, Sm, Eu, Gd, Tb, Dy, Ho, Er, Tm, Yb and Lu.

3. The process of Claim 1 where in the chemical shift reagent is selected from the group consisting of Pr, Nd, Pm, Sm, Tb, Dy, Ho, Er, Tm, Yb and Lu.

4. The process of Claim 1 wherein the chemical shift reagent is selected from the group consisting of Tb, Tm, Pr, Yb and Lu.

5. The process of Claims 1,2,3 or 4 wherein the chemical shift reagent is in an inorganic salt form.

6. The process of Claim 1,2,3 or 4 wherein the chemical shift reagent is in a form complexed with a metal chelator.

7. The process of claim 6 wherein the metal chelator is selected from the group consisting of NTA, EDTA, HETDA, DTPA and N-hydroxyethylimino-diacetic acid.

8. The process of Claim 1,2,3 or 4 wherein the chemical shift reagent is complexed to a target seeking biologically active molecule.

9. The process of Claim 8 wherein the target seeking biologically active molecule is an antibody or antibody fragment.